# EUROPEAN PATENT APPLICATION

(11) **EP 4 520 732 A1**
(43) Date of publication of application: **12.03.2025**
(21) Application number: 23220840.5
(22) Date of filing: 29.12.2023
(51) Int. Cl.: C02F 1/78, A01G 7/06, A01N 25/02, A01N 59/00, A61L 2/18, C02F 103/02, C02F 103/26

(54) **DISINFECTION PLANT FOR A REHYDRATION PROCESS FOR PLANT PORTIONS**

(30) Priority: 11.09.2023 IT 202300018609
(71) Applicant: Nicola, Claudio, 14047 Mombercelli (AT) (IT)
(72) Inventor: Nicola, Claudio, 14047 Mombercelli (AT) (IT)
(74) Representative: Robba, Pierpaolo

(57) **Abstract**

The present invention relates to a disinfection plant (5) for a rehydration process for plant portions of the type including one or more rehydration tanks (3), containing rehydration water, in which said plant portions are immersed. According to the invention, said disinfection plant (5) comprises at least one ozonizing machine (7) suitable for introducing ozone into a portion of said rehydration water, and a hydraulic circuit (9) designed to withdraw said portion of rehydration water from said rehydration tanks (3), convey said portion of rehydration water into said at least one ozonizing machine (7) and redistribute said ozone-treated portion of rehydration water into said one or more rehydration tanks (3). The disinfection plant (5) according to the invention makes it possible to effectively remove pathogens present in the rehydration water and on the plant portions immersed in the rehydration tanks. Said disinfection plant (5) further ensures a disinfecting activity during the entire rehydration process.

## Description

### Technical Field of the Invention

The present invention relates to a disinfection plant used in the process of rehydrating plant portions.

In particular, the present invention relates to a disinfection plant use in a rehydration process that consists of immersing said plant portions in special rehydration tanks containing rehydration water.

### Prior Art

As is known, there exist several techniques of plant propagation.

A popular first technique for agamically propagating fruit plants is grafting.

Grafting means growing on a plant, called rootstock, a plant portion of the same or different species, called scion, in order to obtain a new, more valuable or more productive specimen.

Another popular propagation technique is based on rooting and transplanting cuttings. Cuttings are plant portions that are specially cut from a parent plant and, unlike scions, are placed in soil or water in order to emit roots, thus giving rise to a new individual. Although these two propagation techniques are both based on the use of plant portions, they may involve several steps before the scion is grafted or the cutting is transplanted. As is known, a step common to both these techniques is the rehydration process.

During this process, the plant portions, whether scions or cuttings, are immersed in special rehydration tanks containing water.

Depending on the case, thousands or even tens of thousands of plant portions may be immersed in rehydration tanks during a single rehydration process.

These plant portions, depending on the propagation technique they are intended for and the plant species they belong to, may remain in the rehydration tanks for a few hours or for longer, up to about 24 hours.

It is important to bear in mind that plant portions, whether scions or cuttings, used for plant propagation may contain pathogens (such as viruses, bacteria, fungi or phytoplasmas) that, if not adequately treated, can irreparably damage the contaminated plant portion, rendering it unusable.

Such pathogens cannot always be easily detected with the naked eye.

Consequently, it frequently happens that contaminated plant portions are introduced into the rehydration tanks during the rehydration process.

Once placed in these tanks, the contaminated plant portions can release pathogens into the water, which pathogens, using water as a vehicle, can spread and infect the healthy plant portions, even putting the entire production batch at risk.

Therefore, the rehydration process turns out to be a phase with high biological risk that, if not adequately controlled, exposes not only the plant portions present in the rehydration tanks, but also the biological material that will come into contact with these portions and that will subsequently be used in the production of new plants, to the risk of spreading pathogens.

Notwithstanding the above, the rehydration process is currently a phase in which treatment to ensure disinfection of the water and/or plant portions contained in the rehydration tanks is rarely provided.

This treatment, when implemented, is based on the use of dispensers that, depending on the water flow rate used in the rehydration process, add a certain amount of disinfectant to the water itself.

This type of treatment has a significant limitation in that the disinfectant, being dosed based on the overall volume of water contained in the tanks, cannot ensure continuous disinfectant activity for the entire duration of the rehydration process.

It should also be borne in mind that, at present, the most commonly used disinfectants are hydrogen peroxide and chlorine oxides, which per se have considerable limitations.

Hydrogen peroxide, for example, having a low disinfectant power is not suitable for disinfecting large volumes of water and therefore can hardly be applied to rehydration processes that, as is often the case, involve rehydration tanks of considerable size and capacity.

Chlorine-based compounds, on the other hand, can even be harmful when used to disinfect plants or plant portions. The use of such compounds, in fact, can lead to chlorosis, a disease that occurs when the water that comes into contact with the plant contains an excessive amount of chlorine.

Therefore, it is an object of the present invention to overcome the drawbacks of prior art by providing a disinfection system for a rehydration process that allows effective removal of pathogens present in rehydration water and/or on the plant portions immersed in rehydration tanks.

Accordingly, an object of the present invention is to provide a disinfection system that prevents the spread of pathogens among the plant portions contained in rehydration tanks. In addition, an object of the present invention is to provide a disinfection plant that ensures disinfectant activity throughout the rehydration process.

Finally, an object of the present invention is to provide a disinfection system that allows, by using a special disinfecting agent, a high disinfecting action without damaging the plant portions contained in rehydration tanks.

These and other objects are achieved with a disinfection plant for a rehydration process for plant portions as claimed in the appended claims.

### Summary of the Invention

The present invention relates to a disinfection plant used in the process of rehydrating plant portions, such as scions or cuttings.

The rehydration process for plant portions consists of immersing said plant portions in one or more rehydration tanks containing rehydration water.

Some of these plant portions may contain pathogens that, once introduced in the rehydration tanks together with the plant portions, may disperse within the rehydration water.

According to the invention, a disinfection plant is introduced to prevent such pathogens from using the rehydration water to spread and infect the surrounding plant portions.

Said disinfection plant comprises one or more treatment machines and a hydraulic circuit enabling recirculation of rehydration water so that a rehydration water portion, withdrawn from the rehydration tanks, is conveyed into said one or more treatment machines.

Within said treatment machine, said rehydration water portion is subjected to a disinfection treatment to reduce the quantity of pathogens dispersed therein and, consequently, limit the spread of these pathogens to healthy portions of the plants. According to the invention, at least one of said one or more treatment machines is an ozonizing machine, i.e., a machine that effects the disinfection treatment by using ozone as a disinfecting agent.

As is known, ozone is a gas, composed of three oxygen atoms, with a strong oxidizing effect. This characteristic gives ozone a high disinfectant capacity, superior to that of hydrogen peroxide.

Moreover, as is known, ozone at atmospheric pressure and room temperature is a gas that cannot be stored and must therefore be produced 'in-line', i.e., during the disinfection treatment itself.

Therefore, according to the present invention, said ozonizing machine comprises one or more ozone generators, capable of producing ozone gas, and one or more treatment tanks, connected to said one or more ozone generators.

Through the hydraulic circuit, the rehydration water portion withdrawn from the rehydration tanks is sent to said one or more treatment tanks, in which the ozone produced by said one or more ozone generators is introduced.

Within said treatment tanks, the ozone is injected into the portion of rehydration water. Treatment of disinfection of the portion of rehydration water is thereby obtained, as ozone oxides the pathogens contained in said rehydration water portion.

Advantageously, ozone, being an unstable gas formed from oxygen, reverts to simple oxygen in less than an hour, without leaving any trace in the portion of rehydration water subjected to the disinfection treatment.

This feature differentiates ozone from chlorine-based disinfectants; indeed, ozone, unlike chlorine, can degrade pathogens present in water without releasing by-products.

As both ozone generators and processes allowing ozone injection into a liquid are well known to those skilled in the art, it is not considered necessary to describe them in more detail.

The disinfected portion of rehydration water, exiting the treatment tanks, is redistributed, through the hydraulic circuit, into the rehydration tanks.

Preferably, the withdrawal of the portion of rehydration water from the rehydration tanks and the recirculation of said portion within the hydraulic circuit can be obtained by means of a recirculation device, such as, for example, a recirculation pump.

In addition, both the withdrawal of the rehydration water portion from the rehydration tanks and the redistribution of said portion into the rehydration tanks after the rehydration treatment can be achieved, for example, by exploiting a system of adjustable valves. Advantageously, the disinfection plant of the present invention makes it possible to obtain a double disinfecting action.

The first disinfecting action is achieved by means of the disinfection treatment itself, i.e., by injecting the ozone gas into the rehydration water portion.

The second disinfecting action is achieved by exploiting the already treated portion of rehydration water to bring ozone onto the plant portions immersed in the rehydration tanks, by using the ozone that has not yet turned into oxygen in order to obtain a direct disinfectant effect even on the surface of the plant portions.

As already mentioned, ozone decomposes quickly in water and its duration in aqueous solutions is very short; it is therefore obvious that to exploit the second disinfecting action, it will be necessary to work quickly so that the rehydration water portion in which ozone is dissolved reaches the plant portions immersed in the rehydration tanks.

The production of ozone through ozone generators and the injection of said gas into the rehydration water must be well calibrated to achieve the desired disinfection treatment. As is known, the power of disinfecting water can be assessed by considering the redox potential, measured in milliVolts (mV). The higher the redox potential, the greater the power of oxidizing, and therefore disinfecting, water.

Normally, water that has not undergone any disinfection treatment has a redox potential of less than 200 mV.

To have an effective disinfecting action, the rehydration water portion undergoing disinfection treatment must reach a redox potential of between 500 mV and 1000 mV. This range is influenced by the percentage of dissolved organic material in the hydration water before treatment. This organic material includes plant exudates, material deposited during cultivation and pathogens that are subsequently inactivated by the disinfection treatment itself.

Therefore, ozone gas is produced within the ozone generators and dosed in the rehydration water portion in such a way as to obtain a rehydration water with a redox potential between the two values referred above.

By keeping the redox potential between said values, the disinfection plant of the present invention makes it possible to remarkably reduce the concentration of pathogens contained in the rehydration water portion that has undergone treatment.

In particular, through the disinfection plant of the present invention the quantity of pathogens is reduced from 10³ to 10⁴ times from the original quantity of pathogens, i.e., the quantity of pathogens present before the rehydration water portion was subjected to the disinfection treatment.

This result is obtained with a single disinfection treatment, i.e., through a single passage of the rehydration water portion through the ozonizing machine.

Advantageously, according to another aspect of the present invention, the hydraulic circuit of said disinfection plant allows frequent recirculation, i.e., frequent passages of the portion of rehydration water within at least one of said one or more ozonizing machines in a single rehydration process.

Preferably, said hydraulic circuit allows circulation of the rehydration water portion within at least one of said ozonizing machines from one to ten times per hour during the rehydration process, keeping the water mass under quasi-constant recirculation. Therefore, the disinfection plant of the present invention makes it possible to reduce from one to ten times in a single hour the quantity of pathogens contained in the rehydration water portion withdrawn from the rehydration tanks.

Preferably, according to another aspect of the present invention, upstream of said one or more ozonizing machines, the rehydration water portion can be conveyed, through the hydraulic circuit, into one or more filter devices to remove the suspended parts present in the rehydration water.

In conclusion, the disinfection plant according to the present invention allows disinfection of a rehydration water portion and redistribution of said portion in the rehydration tanks, which actions can be repeated several times during the rehydration process.

This makes it possible to maintain a very low microbiological load in the rehydration tanks and to significantly reduce the biological risk to which plant portions are exposed during the rehydration process.

Therefore, the disinfection plant according to the present invention ensures reasonable biological safety during the rehydration process, by safeguarding, on one part, the nurseryman using said process, and, on the other part, the entire downstream production process.

### Brief Description of Drawings

Further features and advantages of the present invention will become more evident from the ensuing detailed description of a preferred embodiment of the invention, given as a non-limiting example with reference to the annexed figure showing a top view of the plant of the present invention.

### Detailed Description of a Preferred Embodiment of the Invention

In the following description of a preferred embodiment of a disinfection plant for a process of rehydrating plant portions, reference will be made to a disinfection plant used in the process of rehydrating cuttings, more particularly grapevine cuttings intended to become rooted cuttings.

However, this embodiment should not be interpreted in a limiting sense and the disinfection plant according to the invention could be used in the process of rehydrating plant portions of different types, such as, for example, scions or cuttings of other plant species.

A preferred embodiment of the invention will be described below with reference to a rehydration process obtained with a rehydration plant comprising six rehydration tanks with a capacity of about 2700 liters.

This embodiment, however, should by no means be understood as limiting the scope of the invention, which can also be applied to rehydration plants involving a different number of rehydration tanks and/or rehydration tanks with a different capacity.

The rehydration plant incorporating the present invention is indicated as a whole with reference numeral 1, whereas the rehydration tanks are indicated with reference numeral 3. Said rehydration tanks 3 contain rehydration water - usually water provided by the water mains - in which the grapevine cuttings are immersed in any immersion direction. Preferably, said grapevine cuttings are completely immersed in the rehydration tanks 3. As is known, a valve 4 can be installed at one or more of said rehydration tanks 3, said valve opening or closing the water coming from the water mains so as to keep the level of rehydration water within the rehydration tanks within the correct limits.

According to the invention, a disinfection plant 5 is introduced in the rehydration plant 1 to prevent pathogens that may be present on infected cuttings immersed in the rehydration tanks 3 from spreading and infecting the surrounding cuttings, thereby jeopardizing the entire production lot immersed in the tanks.

As illustrated, said disinfection plant 5 mainly comprises an ozonizing machine 7 and a hydraulic circuit 9.

Said hydraulic circuit 9 makes it possible to recirculate a portion of the dehydration water contained in the rehydration tanks 3 so that said rehydration water portion is conveyed into said ozonizing machine 7.

According to an alternative embodiment of the present invention, the disinfection plant 5 may comprise two or more ozonizing machines 7 and the hydraulic circuit 9 may connect one or more of said ozonizing machines 7 to one or more of the rehydration tanks 3.

In the illustrated embodiment, said hydraulic circuit 9 comprises:
- a suction circuit 11 connecting the rehydration tanks 3 to a recirculation pump 13 allowing circulation of said rehydration water portion within the hydraulic circuit 9;
- an intermediate circuit 15 connecting the recirculation pump 13 to the ozonizing machine 7; and
- a delivery circuit 17 connecting the ozonizing machine 7 to the rehydration tanks 3.

When the recirculation pump 13 is activated, a portion of the rehydration water contained in the rehydration tanks 3 is conveyed into the suction circuit 11, flows through the circulation pump 15 and is introduced into the ozonizing machine 7, inside which it is subjected to a disinfection treatment.

Once such treatment has been completed, said rehydration water portion is redistributed, by means of the delivery circuit 17, to the rehydration tanks 3.

In more detail, the suction circuit 11 comprises, for each rehydration tank 3, a suction line 19, directly connected to the rehydration tank 3, and a suction valve 21.

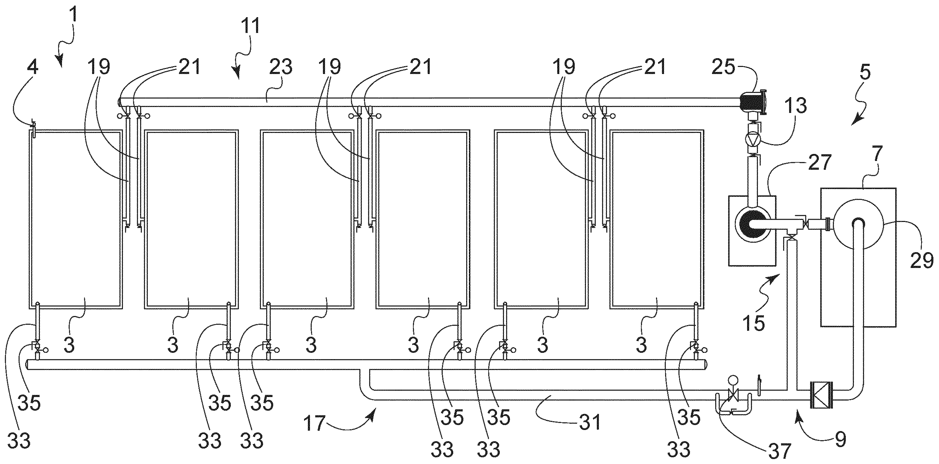

Preferably, the suction valves 21 are motorized valves 2" made of stainless steel AISI 316. Said suction duct 11 further comprises a collector pipe 23 upstream of which the suction lines 19 are connected and downstream of which the recirculation pump 13 is connected, as can be seen from the figure.

In a preferred embodiment of the invention, a first filter device 25, also visible in the figure, can be mounted upstream of the recirculation pump 13 and at the collector pipe 23, said first filter device being suitable for filtering any impurities that may be present in the rehydration water portion withdrawn by the recirculation pump 13.

Said first filtering device 25 can be, for example, a filter made of stainless steel and provided with a pull-out basket, sized according to the water flow rate flowing within the suction circuit 11 and the impurities present in the rehydration water.

In an even more preferred embodiment of the invention, a second filter device 27 may be provided, arranged on the intermediate circuit 15, between the recirculation pump 13 and the ozonizing machine 7, said rehydration water portion flowing through said second filter device before reaching the ozonizing machine 7.

The intermediate circuit 15 may further comprise, upstream of the ozonizing machine 7, a by-pass duct which makes it possible to convey the rehydration water portion withdrawn by the recirculation pump 13 towards other machines, devices, or plants, to subject said water portion to different treatments.

Therefore, according to the preferred embodiment described above and shown in the figure, when the recirculation pump 13 is running, a certain amount of rehydration water is withdrawn from each rehydration tank 3 and, flowing through the corresponding suction line 19 and the corresponding suction valve 21, flows into the collector pipe 23, said amounts of rehydration water together forming the portion of rehydration water withdrawn by the recirculation pump 13.

Such water portion is filtered by the first filter device 25, flows through the recirculation pump 13, is passed through the second filter device 27 and is subsequently transferred into the ozonizing machine 7.

Said ozonizing machine 7 allows subjecting the rehydration water portion to a disinfection treatment, using ozone as disinfecting agent.

Said ozonizing machine 7 comprises one or more ozone generators (not shown), capable of producing ozone gas, and a treatment tank 29, connected to said one or more ozone generators.

Alternatively, said ozonizing machine 7 may comprise one or more treatment tanks 29, each connected to one or more ozone generators.

During operation of the recirculation pump 13, when the portion of rehydration water reaches the ozonizing machine 7, said water portion is transferred into the treatment tank 29.

The ozone produced by said one more ozone generators is also introduced into said treatment tank 29, which ozone is injected into the rehydration water portion present in the tank.

In this way, disinfection treatment of the portion of rehydration water is obtained, as ozone has an oxidizing action on the pathogens dispersed therein.

Since both ozone generators and the processes that allow ozone to be injected into a liquid are well known to those skilled in the art, it is not considered necessary to describe them in more detail.

Once the disinfection treatment within the treatment tank 29 has been completed, the rehydration water portion is redistributed, through the delivery circuit 17, to the rehydration tanks 3.

In particular, the delivery circuit 17 shown in the Figure comprises a distribution pipe 31 transferring the portion rehydration water into delivery pipes 33, one for each rehydration tank 3.

Said delivery pipes 33, by means of delivery valves 35, redistribute the treated portion of rehydration water to the rehydration tanks 3.

Preferably, the suctions valves 35 are motorized valves made of stainless steel AISI 316.

In a preferred embodiment of the invention, a motorized valve 37 is provided at the distribution pipe 31 of the delivery circuit 17, said valve, combined with a pressure transducer, allowing keeping the steady-state pressure inside the ozonizing machine 7 when the recirculation pump 13 is running.

Preferably, monitoring and adjustment of the disinfection plant 5 of the present invention can be managed through a control cabinet.

By acting onto the selector switches of the control cabinet the operator can, for example, connect or isolate every single rehydration tank 3 with respect to the hydraulic circuit 9. Advantageously, the disinfection plant of the present invention allows to obtain a double disinfecting action.

The first disinfecting action is achieved by means of the disinfection treatment itself, i.e., by injecting the ozone gas into the rehydration water portion during the disinfection treatment within the ozonizing machine 7.

The second disinfecting action is achieved by exploiting the already treated portion of rehydration water to bring ozone onto the grapevine cuttings immersed in the rehydration tanks 3, thereby obtaining direct disinfection even on the surface of said cuttings.

The production of ozone, by means of said one or more ozone generators, and the injection of said gas into the rehydration water portion must be well calibrated to obtain this double disinfecting action.

If the ozone concentration in the rehydration water portion is too low, the disinfection treatment does not reach a significant reduction of the pathogens contained in said portion. If the ozone concentration in the rehydration water portion is too high, there is a risk that said portion, one reintroduced into the rehydration tanks 3, causes damage to those plant portions with which it comes into contact.

As is known, the ozone concentration must be a function of the amount of rehydration water that undergoes treatment as well as of the content of organic matter present in said amount.

According to the present invention, the ozonizing machine 7 makes it possible to produce a quantity of ozone such that a concentration of ozone in the rehydration water portion between 0.2 e 0.9 mg/l, preferably between 0.4 e 0.6 mg/l, is obtained, with a treatment time (defined as the time the rehydration water portion undergoing treatment remains inside the treatment tank 29) between 1 and 10 minutes, preferably between 3 and 7 minutes.

The values referred above make it possible to break down the main pathogens in water, without damaging the plant portions.

In particular, the disinfection plant 5 of the present invention makes it possible to reintroduce, into the rehydration tanks 3, a rehydration water portion which has a redox potential between 500 mV and 1000 mV and in which, accordingly, the quantity of pathogens is reduced from 10³ to 10⁴ times compared to the original quantity of pathogens, i.e., compared to the quantity of pathogens present before the rehydration water portion was subjected to the disinfection treatment.

As far as the second disinfecting action is concerned, it should also be taken into consideration that ozone, being an unstable gas formed from oxygen, decays very quickly, especially in water, reverting to simple oxygen.

The temperature of the rehydration water has an important influence on the ozone decay time: if the water temperature is 15°C, ozone decays in approximately 30 minutes, if the water temperature is 20°C, ozone decays in approximately 20 minutes, leaving no trace in the rehydration water portion subjected to the disinfection treatment.

Therefore, the hydraulic circuit 9 must allow, once the disinfection treatment has been completed, rapid redistribution of the rehydration water portion to the rehydration tanks 3, before the ozone dissolved in that portion decays completely.

In particular, the hydraulic circuit 9 of the present invention allows the portion of rehydration water that has undergone treatment to be redistributed in less than 30 minutes, preferably in less than 15 minutes, thereby achieving a second effective disinfecting action.

By complying with the above parameters, it is therefore possible to exploit the oxidative capacity of ozone to break down pathogens, both biological and chemical, present not only in the rehydration water, but also on the surface of the plant portions subjected to the rehydration process.

In particular, the biocide capacity of ozone can be applied against viruses, such as the Tomato ring spot, bacteria, such as Xylella fastidiosa, fungi, such as the mycetes precursors of the grapevine esca disease and phytoplasmas, such as the flavescence dorée phytoplasma.

In addition, advantageously, the hydraulic circuit 9 of the disinfection plant 5 of the present invention makes it possible to withdraw portions of rehydration water and convey them into the ozonizing machine 7 several times during the rehydration process, thereby increasing both the effect provided by the first disinfecting action and the effect provided by the second disinfecting action.

This result can be achieved by activating the recirculation pump 13 several times during the rehydration process.

Preferably, said hydraulic circuit 9 makes it possible to circulate the rehydration water portion inside the ozonizing machine 7 from one to ten times per hour, keeping the water mass in quasi-constant recirculation.

Preferably, the hydraulic circuit 9 of the present invention is structured in such a way as to allow, within one hour, to subject to disinfection treatment, i.e., to circulate inside the ozonizing machine 7, an amount of water equal to at least 70% of the quantity of overall rehydration water contained in the rehydration tanks 3.

In particular, the hydraulic circuit 9 of the disinfection plant 5 of the present invention can be programmed to alternate one or more activation periods and one or more stop periods, obtained, preferably, by activating or switching off the recirculation pump 13.

According to a preferred embodiment of the invention, each activation period, comparable to the period during which the rehydration water portion is withdrawn and circulated inside the ozonizing machine 7, can be between 5 and 40 minutes, preferably between 10 and 30 minutes, and each stop period, during which no rehydration water portion is withdrawn, can be between 5 and 40 minutes, preferably between 10 and 20 minutes. Moreover, the hydraulic circuit 9 of the present invention can be structured in such a way as to achieve a total duration of the disinfection treatment corresponding to the sum of said one or more activation periods, corresponding to 10% - 40% of the duration of the entire rehydration process, preferably corresponding to 15% - 25% of the duration of the entire rehydration process.

In addition, the disinfection plant 5 according to the present invention can be equipped with a pause/work timer and an automatic switching off system where the time can be set by the operator.

Therefore, the disinfection plant 5 according to the present invention makes it possible to disinfect a portion of rehydration water and to redistribute said portion into the rehydration tanks, said actions being repeatable several times during the rehydration process. Accordingly, the disinfection plant 5 according to the present invention allows continuous disinfection of the rehydration water contained in the rehydration tanks 3.

This remarkably limits the spread of pathogens among the grapevine cuttings immersed in the tanks during the rehydration process.

The disinfection plant according to the present invention therefore ensures remarkable biological safety during the rehydration process, safeguarding, on one part, the nurseryman using this process and, on the other part, the entire downstream production process using the plant portions subjected to the rehydration process.

It will be evident to the person skilled in the art that the embodiments described above in detail are in no way to be understood in a limiting sense, and that numerous variations and modifications are possible without going beyond the scope of the invention as defined in the appended claims.

## Claims

1. Disinfection plant (5) for a rehydration process for plant portions of the type including one or more rehydration tanks (3), containing rehydration water and suitable for receiving said plant portions, said disinfection plant being **characterized in that** it comprises:
- one or more treatment machines, wherein at least one of said treatment machines is an ozonizing machine (7) suitable for introducing ozone into a portion of said rehydration water;
- a hydraulic circuit (9) designed to withdraw said portion of rehydration water from said rehydration tanks (3), convey said portion of rehydration water into said at least one ozonizing machine (7) and redistribute said ozone-treated portion of rehydration water into said one or more rehydration tanks (3).

2. Disinfection plant (5) according to claim 1, wherein said ozonizing machine (7) comprises one or more ozone generators, suitable for producing ozone gas, and one or more treatment tanks (29), connected to said one or more ozone generators, and wherein said one or more treatment tanks (29) are suitable for receiving therein the ozone produced by said one or more ozone generators and said rehydration water portion withdrawn from said hydraulic circuit (9).

3. Disinfection plant (5) according to claim 1, wherein said hydraulic circuit (9) is suitable for withdrawing said portion of rehydration water from said rehydration tanks (3), conveying said portion of rehydration water into said at least one ozonizing machine (7) and redistribute said ozone-treated portion of rehydration water into said one or more rehydration tanks (3) from one to ten times per hour during the rehydration process.

4. Disinfection plant (5) according to claim 3, wherein said hydraulic circuit (9), within an hour, is suitable for withdrawing and conveying into said at least one ozonizing machine (7) a quantity of water equal to at least 70% of the quantity of overall rehydration water contained in said one or more rehydration tanks (3).

5. Disinfection plant (5) according to claim 1, wherein said hydraulic circuit (9) is suitable for redistributing said ozone-treated portion of rehydration water, exiting said ozonizing machine (7), into said one or more rehydration tanks (3) in less than 30 minutes.

6. Disinfection plant (5) according to claim 5, wherein said hydraulic circuit (9) is suitable for redistributing said ozone-treated portion of rehydration water, exiting said ozonizing machine (7), into said one or more rehydration tanks (3) in less than 15 minutes.

7. Disinfection plant (5) according to any of the previous claims, wherein said hydraulic circuit (9) comprises a recirculation device suitable for circulating said portion of rehydration water inside said hydraulic circuit (9), and wherein said recirculation device is a recirculation pump (13).

8. Disinfection plant (5) according to claim 2, wherein said at least one ozonizing machine (7) is suitable for producing a quantity of ozone such that an ozone concentration between 0.2 and 0.9 mg/l is obtained in said portion of rehydration water, with a treatment time between 1 and 10 minutes, the treatment time being the time over which said portion of rehydration water remains inside said one or more treatment tanks (29).

9. Disinfection plant (5) according to claim 8, wherein said at least one ozonizing machine (7) is suitable for producing a quantity of ozone such that an ozone concentration between 0.4 and 0.6 mg/l is obtained in said portion of rehydration water, with a treatment time between 3 and 7 minutes, the treatment time being the time over which said portion of rehydration water remains inside said one or more treatment tanks (29).

10. Disinfection plant (5) according to claim 3, wherein said hydraulic circuit (9) can be programmed to alternate one or more activation periods, i.e., periods in which the portion of rehydration water is withdrawn and circulated inside said ozonizing machine (7), and one or more stop periods, i.e., periods in which no portion of rehydration water is withdrawn, and wherein each of said one or more activation periods is between 5 and 40 minutes and each of said one or more stop periods is between 5 and 40 minutes.

11. Disinfection plant (5) according to claim 10, wherein each of said one or more activation periods is between 10 and 30 minutes, and wherein each of said one or more stop periods is between 10 and 20 minutes.

12. Disinfection plant (5) according to claim 10, wherein the total duration of the disinfection treatment, corresponding to the sum of said one or more activation periods, is 10% - 40% of the duration of the entire rehydration process.

13. Disinfection plant (5) according to claim 10, wherein the total duration of the disinfection treatment, corresponding to the sum of said one or more activation periods, is 15% - 25% of the duration of the entire rehydration process.

14. Disinfection plant (5) according to claim 7, wherein said hydraulic circuit (9) comprises:
- a suction circuit (11) suitable for connecting said rehydration tanks (3) to said recirculation pump (13);
- an intermediate circuit (15) suitable for connecting said recirculation pump (13) to said ozonizing machine (7), wherein said intermediate circuit (15) includes a bypass suitable for conveying said portion of rehydration water towards other machines, devices or plants;
- a delivery circuit (17) suitable for connecting said ozonizing machine (7) to said rehydration tanks (3), wherein, at said delivery circuit (17), a motorized valve (37) is provided suitable for maintaining a certain level of pressure inside said ozonizing machine (7).

15. Process of rehydrating plant portions by means of a disinfection plant comprising one or more rehydration tanks (3), containing rehydration water and suitable for receiving said plant portions, one or more treatment machines, wherein at least one of said treatment machines is an ozonizing machine (7) suitable for introducing ozone into a portion of said rehydration water, a hydraulic circuit (9) designed to withdraw a portion of rehydration water from said at least one rehydration tank (3), convey said portion of rehydration water into said at least one ozonizing machine (7) and redistribute said ozone-treated portion of rehydration water into said one or more rehydration tanks (3), wherein the following steps are provided:
- by means of at least one of said treatment machines, introducing ozone into a portion of said rehydration water;
- by means of said hydraulic circuit:
- withdrawing a portion of said rehydration water from said at least one rehydration tank;
- conveying said portion of said rehydration water into said at least one ozonizing machine (7);
- redistributing said ozone-treated portion of rehydration water into said at least one rehydration tank (3).
